(19) Europäisches Patentamt
European Patent Office
Office européen des brevets

(11) **EP 4 431 970 A1**

(12) **EUROPEAN PATENT APPLICATION**

(43) Date of publication:
**18.09.2024 Bulletin 2024/38**

(21) Application number: **24162192.9**

(22) Date of filing: **07.03.2024**

(51) International Patent Classification (IPC):
**G01R 33/565** (2006.01)    **G01R 33/561** (2006.01)
**G01R 33/56** (2006.01)

(52) Cooperative Patent Classification (CPC):
**G01R 33/5659; G01R 33/5608; G01R 33/5612**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **09.03.2023 CN 202310226348**

(71) Applicant: **Siemens Healthineers AG
91301 Forchheim (DE)**

(72) Inventors:
• **Herrler, Jürgen
91058 Erlangen (DE)**
• **Liebig, Patrick
91052 Erlangen (DE)**

(74) Representative: **Siemens Healthineers
Patent Attorneys
Postfach 22 16 34
80506 München (DE)**

(54) **METHOD AND MRI SYSTEM FOR CALCULATING AT LEAST ONE OPTIMIZED INITIAL B1-SHIM FOR A MAGNETIC RESONANCE MEASUREMENT**

(57) The invention relates to a method for calculating a set of optimized initial B1-shims (12) for an MR measurement, wherein a B1-shim comprises a vector of complex B1-shim coefficients, each coefficient representing a scaling factor for one element of a multi-element transmit coil, the method comprising the steps of: (a) receiving a set of previously measured B1-maps (6) for one or several body parts of various test subjects, (b) calculating a set of B1-shims (8) for a plurality of different field-of-views in the one or several body part(s) by means of an optimization algorithm, (c) identifying which B1-shim has the best performance for a group (35) of field-of-views by using the previously measured B1-maps, and (d) optimizing the B1-shim for that group of field-of-views to obtain an optimized initial B1-shim (12)p. The invention also relates to a computer program, a B1 shim design unit, a control unit and an MRI system.

FIG 4

**Description**

In magnetic resonance imaging (MRI) at "ultra-high" magnetic field strengths of the B0-field (ultra-high field MRI, e.g. at more than 5T, in particular at 7T or more, the radio frequency (RF) excitation field, also referred to as B1-field, which is generated by the transmit RF coil, becomes spatially strongly inhomogeneous, mainly due to the short Larmor wavelength. When using multi-element transmit coils, e.g., phased array RF coils, a technique called "Parallel Transmit" (pTx) may be used to compensate for these inhomogeneities. Here, several excitation coils are driven each with its own radio frequency (RF) pulse shape. The resulting excitation field arises from the interference of the fields generated by the individual coils and can thus be controlled in its spatial distribution.

Static pTx, also called "B1-shimming", consists of driving all coils with the same RF pulse shape (e.g., square or sinc), but scaling it with coil-specific magnitudes and phases. This produces an excitation field that can be more spatially homogeneous than an uncalibrated excitation field. In 2D sequences, only one slice in the head is excited, and with clever adjustment of magnitudes and phases, significantly better field distributions can be generated especially in this case than in the uncalibrated case (usually: circularly polarized B1-field, regardless of the patient or the layer to be excited).

To adjust these magnitudes and phases so that the resulting magnetization in the layer to be excited becomes as spatially homogeneous as possible, a non-convex optimization problem based on the B1-fields of the individual transmitting coils must be solved. In addition, the B1-fields of the individual transmitting coils are dependent on the anatomy of the individual patient, which is why this adjustment can only be performed during the examination. Thus, the optimization must be performed quickly, but still function robustly and for a wide variety of Bl-fields. To calculate the best possible combination of amplitudes and phases of the transmitting channels (also called: "B1-shim"), a minimization problem is formulated, which may for example be solved with gradient descent methods under certain constraints, such as maximum voltage at a transmitting channel, and limited Specific Absorption Rate (SAR) exposure. For example, inner-point methods, as described in K. Majewski "Simultaneous optimization of radio frequency and gradient waveforms with exact Hessians and slew rate constraints applied to kT-points excitation", Journal of Magnetic Resonance, Volume 326, 2021, 106941, ISSN 1090-7807) or active-set methods, as described in A. Hoyos-Idrobo, P. Weiss, A. Massire, A. Amadon and N. Boulant, "On Variant Strategies to Solve the Magnitude Least Squares Optimization Problem in Parallel Transmission Pulse Design and Under Strict SAR and Power Constraints", in IEEE Transaction on Medical Imaging, vol. 33, no. 3, pp. 739-748, March 2014) may be used. Furthermore, a very successful and established algorithm was presented by Setsompopp et al. in K. Setsompop ,L.L. Wald, V. Alagappan, B.A. Gagoski, E. Adalsteinsson "Magnitude least squares optimization for parallel radio frequency excitation design demonstrated at 7 Tesla with eight channels". Magn. Reson. Med. 2008; 59(4):908-915.

However, all of these algorithms are by default initialized with a Bl-shim having constant amplitudes and phases, which produce a circularly polarized B1 field. This often leads to suboptimal results, since the problem to be solved is non-convex. Thus, the optimization algorithm may end up not in the absolute minimum, but in a suboptimal local minimum.

A well-known problem is the appearance of "holes" in the resulting magnetization distributions, i.e. the resulting B1-field magnitude in a small local area within the imaging slice is zero or almost zero, so that the resulting MR signal is also zero or close to zero. This is particularly undesirable, since with zero signal amplitude, no diagnosis is possible in this area.

Therefore, the object of the invention is to find a method which allows improved B1-shimming, as well as a related computer program and magnetic resonance imaging system. In particular, it is an object of the invention to provide an improved method for B1-shimming which reliably avoids the above-identified disadvantages of having "holes" in the magnetization distribution, and therefore in the magnetic resonance (MR) image.

These objects are met or exceeded by a method for calculating at least one optimized initial Bl-shim, a computer program according to claim 11, a non-transient computer-readable medium according to claim 12, a Bl-shim design unit according to claim 13, a control unit for a magnetic resonance imaging system according to claim 14, and an MRI system according to claim 15.

According to a first aspect of the invention, a method is provided for calculating at least one optimized initial B1-shim for a magnetic resonance measurement, wherein a Bl-shim comprises a vector of complex Bl-shim coefficients, each coefficient representing a scaling factor for one element of a multi-element transmit coil which is to be used in the magnetic resonance measurement, the method comprising the steps of: (a) receiving a set of previously measured Bl-maps of the multi-element transmit coil for one or several body parts of various test subjects, (b) calculating a set of Bl-shims for a plurality of different field-of-views in the one or several body part(s) of the various test subjects from the previously measured Bl-maps by means of an optimization algorithm, (c) identifying which Bl-shim has the best performance for a group of field-of-views by using the previously measured B1-maps, and (d) optimizing the Bl-shim for that group of field-of-views to obtain an optimized initial Bl-shim.

The invention has recognized that better Bl-shims can be reached by using better-fitting initial shims, which are referred to as optimized initial Bl-shims. The method has been devised to overcome the difficulty, that the optimization problem of B1-shimming is non-convex, thus if the starting values are unsuitable, the optimization algorithm can end up in an unfavourable local minimum. Therefore, the invention aims at using a favourable starting point for the B1-shimming optimization.

The method of claim 1 may be performed once for each multi-element transmit coil and/or for each magnetic resonance imaging system, i.e., it does not have to be repeated for each MR measurement. It may also be carried out only once by the manufacturer of the MRI system or the multi-element transmit coil, and the result, namely one or preferably a set of optimized initial Bl-shims, may be stored in a digital storage to which the MRI system has access to. The method may be used for optimal B1-shimming within a certain field-of-view within a body part of a subject. However, it may also be used to find optimal Bl-shims e.g. for RF saturation pulses which are to excite or suppress the spins outside of an imaging field-of-view.

According to an embodiment of the invention, a method is provided to select one of the sets of optimized initial Bl-shims during each MR measurement. This optimized initial Bl-shim may then be used as the starting point for the online-optimization (during the MR measurement). In this optimization, the specific absorption rate (SAR) limits for the complete magnetic resonance measurement, in particular for the MR sequences to be used, may be taken into account. In particular, several different optimized initial Bl-shims may be used for different field-of-views.

In the following, a field-of-view may mean a region of interest within a body part of the subject which is to be excited by an RF pulse, e.g., a particular slice or slab through the body part, which may be in sagittal, coronal or transversal orientation, or any orientations in between. The field-of-view may also be a part of such slice or slab, e.g., only the inner part, when the outer region is not of interest for the MR measurement. The MR measurement may be any magnetic resonance imaging or spectroscopy measurement which is to be carried out during an examination of a subject, wherein the subject may be a human or animal subject, in particular a patient. The body part may be any body part on which an MR measurement is to be carried out, e.g., a head, neck, a limb, part of a limb like a knee, hand, foot, thorax, abdomen, or parts thereof such as an inner organ, e.g., the liver, heart, kidney, bowels, etc.

A Bl-shim, which may also be referred to as RF-shim or RF-shim setting, comprises a vector of complex Bl-shim coefficients, each coefficient representing a scaling factor for one element of a multi-element transmit coil, which is to be used in the magnetic resonance measurement. This coefficient is usually complex, to take into the account not only the magnitude, but also the phase of the RF pulse. A multi-element transmit coil may be a local radio frequency (RF) coil, which is placed close to the body part, e.g., a phased-array coil. The Bl-shim is in particular a static Bl-shim. In other words, the Bl-shim coefficients are used to scale the RF power delivered by each element of the RF coil, but each coil is driven with the same pulse shape.

The method comprises a first step (a) of receiving a set of previously measured Bl-maps of the multi-element transmit coil for one or several body parts of various test subjects. A Bl-map, also referred to as B1 field sensitivity map, is thus the Bl-field distribution of each element/channel of the transmit coil. Thus, for each body part in each test subject, as many Bl-maps are measured as there are elements in the multi-element transmit coil. The B1 field distribution of all elements together are referred to as "B1-map" herein. Because the Bl-field has magnitude and phase, it is a complex map, also referred to as B1+ map. The complex Bl-maps may be obtained for example with a hybrid B1+ mapping technique as described in Van de Moortele P.F. et al. "Calibration tools for RF shim at very high field with multiple element RF Coils: from ultra fast local relative phase to absolute magnitude B1+ mapping", Proceedings of the 15th Annual Meeting of ISMRM, Berlin, 2007, Abstract No. 1676. This method comprises merging one absolute large flip angle |B1+| map measured with all coils transmitting together in a known B1 shim setting, with $N_{Tx}$ complex, relative B1+ maps derived from small flip angle, multi-slice gradient echo images acquired with only one Tx element transmitting at a time. Other methods for obtaining subject-specific B1 field sensitivity maps are described for example in the Thesis by Kawin Setsompop "Design Algorithms for Parallel Transmission in Magnetic Resonance Imaging", Massachusetts Institute of Technology, 2008, which is incorporated herein by reference.

The B1-shimming problem therefore essentially consists in finding the Bl-shim, thus the vector of complex Bl-shim coefficients, which allows to combine the Bl-fields of the several elements of the RF coil, so that the overall Bl-field is as homogeneous as possible. This optimization is done in step (b) and in several other method steps of various embodiments of the invention by means of an optimization algorithm. This optimization algorithm may use a static B1 shimming technique, in other words the RF shim settings are constant during the RF pulse. A static RF shimming method is for example described in Mao W, Smith MB, Collins CM. "Exploring the limits of RF shimming for high-field MRI of the human head", Magn. Reson. Med. 2006; 56(4): 918-922. With this technique, only the overall amplitude and phase and not the shape of the RF pulse are allowed to vary from transmit element to transmit element. In order to excite a slab, a sinc pulse with different amplitude and phase may be applied to each transmit element. The optimization algorithm may use a gradient descent method, in particular an inner-point method, as disclosed in the above-cited article by Majewski. Circularly polarized Bl-fields may be used as starting points for this optimization algorithm. However, other optimization algorithm known in the art may be used in this step.

The set of previously measured Bl-maps may comprise either Bl-maps of the same kind of body part, e.g., only of the head of various test subjects. It may also comprise Bl-maps of a variety of body parts of various test subjects, e.g., a variety including Bl-maps measured for heads, shoulders, knees, thorax, etc. The Bl-maps have been measured on various test subjects using known methods of measuring the Bl-maps of a multi-element transmit coil. Such measurements are usually performed at the beginning of each MR measurement which requires B1-shimming.

Step (b) comprises calculating a set of Bl-shims for a plurality of different field-of-views, using the Bl-maps for the one or several body parts of the various test subjects. The different field-of-views may for example correspond to different slices or slabs through the body part, e.g., a stack of slices in one particular orientation. This is done because the required Bl-shim may be different for a different field-of-view within in the same body part, because of the severe inhomogeneity of the Bl-maps of each element of the transmit coil.

These Bl-shims, although they have been optimized, may still not provide the ideal Bl-shim for each field-of-view, for the reasons described above. Therefore, the method comprises a further step (c) of identifying which Bl-shim has the best performance for a group of field-of-views, by using the previously measured Bl-maps. In this step, Bl-shims which have been calculated for field-of-views in one body part, e.g., the head, may be applied to completely different body-parts, to find out whether they may also give satisfactory results. A performance index may then be calculated, and groups of field-of-views with similar performance may be formed. This may be done by a clustering method. It is advantageous to find Bl-shims which perform well for a group of field-of-views, in order to find a set of potentially favorable initial Bl-shims, which may be used as a starting point for an optimization algorithm during an actual MR measurement. To further improve the Bl-shim, which has been identified as having the best performance for a group of field-of-views, the Bl-shim is optimized for that group of field-of-views to obtain an optimized initial Bl-shim. Also in this step (d), an optimization algorithm using a gradient descent method, e.g., an inner-point method as disclosed by Majewski, may be used, or a B1-shimming method as described in Mao W et al..

According to an embodiment, the method comprises the steps of

(c1) clustering the Bl-shims calculated for the plurality of different field-of-views and identifying an average Bl-shim for each cluster;

(c2) applying the average Bl-shims to each of the plurality of field-of-views and calculating a performance index for each combination of average Bl-shim and field-of-view using the previously measured Bl-maps;

(c3) clustering the field-of-views according to their performance indices to identify groups of field-of-views which perform similarly well with a similar Bl-shim, and

(d) for each group of field-of-views, optimizing the Bl-shim to obtain an optimized initial Bl-shim.

The Bl-shims which have been optimized for the plurality of different field-of-views may be clustered according to their similarity, so that Bl-shims which have similar coefficients form one cluster. Once the clusters have been defined, the Bl-shims of each cluster may be averaged to calculate an average Bl-shim. The average may for example be the arithmetic mean, the median or any other suitable type of average for the cluster. The average Bl-shim may also be the midpoint or centre of gravity of each cluster. The idea behind step c1 is that the average Bl-shims together define a space where possible suitable shims may be found.

In a next step c2, these average Bl-shims are then applied to each of the plurality of field-of-views, and a performance index is calculated for each combination of average Bl-shim and field-of-view, using the previously measured Bl-maps for each field-of-view in a body part. Thereby, Bl-shims which have been optimized for one subject, may now be used on the same or a different field-of-view in another subject. To keep the calculation effort in this step within a certain limit, it is helpful to only apply the average Bl-shims. In this step, "apply" may mean that the average Bl-shim is used as the starting point of an individual optimization for that field-of-view. The performance index indicates whether the average Bl-shim results in a homogeneous magnetization distribution when applied to the particular field-of-view. The performance index may include a comparison of a target flip angle with an actual flip angle which will be achieved when using the Bl-shim with the previously measured Bl-maps.

In a next step c3, also the field-of-views are clustered according to their performance indices in order to identify groups of field-of-views which perform similarly well with a similar Bl-shim. The clustering preferably identifies groups of field-of-views, wherein not all field-of-views are part of a group, but only those which are close to the average or centre of gravity of each cluster.

Once these clusters have been identified, a Bl-shim is optimized for each cluster or group of field-of-views to obtain an optimized initial Bl-shim. In a useful embodiment, this optimization uses a number of different Bl-shims as staring point, in particular the Bl-shims which have been optimized for the different field-of-views in step (b), and evaluating the performance of each resulting Bl-shim, for example by comparing the resulting magnetization distribution with the target magnetization distribution in the group of field-of-views. The resulting Bl-shim which has the best performance for that group of field-of-views is then taken as an "optimized initial Bl-shims".

The different clusters in the second feature space represent different B1s, for which the MR system has to be able to perform robust B1-shimming. By optimizing the Bl-shim for several field-of-views at a time, more robust shims can be found then for an individual optimization, in which for example the above-mentioned "holes" can be produced.

Therefore, one can also use the method of the invention to create B1-shimming for universal pulses, e.g. saturation pulses.

According to an embodiment, the steps of performing a clustering of the Bl-shims and identifying an average Bl-shim for each cluster may be performed by (c1.1) representing the set of Bl-shims calculated in step (b) in a first feature space, wherein the dimensions of the feature space are the complex shim coefficients of each Bl-shim, (c1.2) performing a cluster analysis of the Bl-shims in the first feature space and calculating a midpoint of each cluster, each midpoint being the average Bl-shim for that cluster.

Thus, the feature space in which the set of Bl-shims are arranged, may have twice as many dimensions as there are elements in the multi-element RF coil, or as the number of transmit channels of the RF coil. In this first feature space, each Bl-shim is represented by one data point. The clustering may be performed using a known cluster analysis algorithm, such as k-means clustering. According to an embodiment, a useful number of clusters may be found by calculating the within-cluster-sum-of-squares (WCSS) value as a function of the number of clusters/classes of shims. According to an embodiment, between 20 and 1000, preferably between 40 and 500, more preferably between 60 and 200 Bl-shims may be calculated in step b, i.e., so many different field-of-views in the one or several body parts of the various test subjects may be used. The WCSS value is a measure for how similar the Bl-shims within one cluster are. If the WCSS value has a sharp drop at a certain number of clusters, then this number of clusters is probably suitable. However, if there is no such sharp drop, the user may have to choose the number of clusters, or the number of clusters may be pre-determined. For example, it may be determined that between 5 and 30, preferably between 10 and 20 Bl-shims are to form one cluster. Alternatively, one may also determine the number of clusters that are desirable for the different field-of-views, e.g., between 10 and 40, preferably between 20 and 30 clusters.

In a next step, the midpoint of each cluster is to be determined. This may be the centre of gravity of each cluster, e.g., the arithmetic mean or any other kind of mean. The midpoint or centre of gravity is then defined as the average B1-shim for that cluster.

According to an embodiment, the steps of clustering the field-of-views according to their performance indices and optimizing the Bl-shim to obtain an optimized initial Bl-shim for this group of field-of-views are performed by (c3.1) representing the performance indices calculated in step (c2) in a second feature space, in which each field-of-view is represented by one data point, and the dimensions of the feature space are the performance indices of each average Bl-shim; (c3.2) performing a cluster analysis on the second feature space, and determining the group of field-of-views which are closest to the centre of each cluster; (d.1) for each group of field-of-views, calculating an optimized Bl-shim using an optimization algorithm; (d.2) providing the optimized B1-shims as optimized initial Bl-shims for the field-of-views within the group of field-of-views.

Thus, the performance indices calculated for each field-of-view are represented in a second feature space, wherein the dimensions of the feature space are the performance indices of each average Bl-shim. Thus, each field-of-view is represented by one data point. The performance index may be the root-mean-square-error (RMSE), i.e., a value obtained by simulating a flip angle distribution or magnetization distribution for each voxel in the field-of-view from the Bl-maps and the average Bl-shim, comparing this value for each voxel with the target flip angle or target magnetization, taking the square of the deviation, and averaging this square for all voxels within the field-of-view. One may divide this value by the square of the target flip angle or target magnetization, respectively, in order to obtain a normalized root-mean-square-error, NRMSE. This value is the performance index of each combination of field-of-view and Bl-shim. The respective feature space thus has a number of dimensions which is the same as the number of average Bl-shims.

In a next step, a cluster analysis may be performed on the second feature space, in order to determine the group of field-of-views which are closest to the centre of each cluster. The clustering may again be performed using k-means clustering. Thereby, the different field-of-views may be classified into different clusters. Thereby, those field-of-views or slices which perform similarly well for similar shims, may be identified. As for the first feature space, one may calculate the WCSS values for different numbers of clusters, and thereby identify a suitable number. Alternatively, the number of clusters may be pre-determined, e.g., that between 10 and 50, preferably between 15 and 30 field-of-views should be within each cluster.

By performing the clustering in the second feature space, one may find out, which field-of-views may advantageously be grouped together in order to find one suitable initial B1-shim.

Finally, the optimized initial Bl-shims are now optimized for each cluster. According to an embodiment, the Bl-shim is only optimized for those field-of-views which are closest to the centre of gravity of each cluster, whereby for example the Euclidean distance may be used. The centre of gravity of each cluster may be calculated as described above, e.g., by calculating the arithmetic mean. Different clusters represent different B1 field distributions, for which one has to optimize during the MR measurement.

All method steps described so far may be performed offline, i.e., using previously measured Bl-maps which may have been obtained from a variety of test subjects at an earlier time. Therefore, the method according to the first aspect of the invention is computer-implemented and may be performed on any kind of computer or processing unit, e.g., on a CPU or GPU of a computer, such as a PC, laptop, smartphone, tablet computer, cloud computer, etc.

According to an embodiment, the clustering of the Bl-shims in the first feature space and/or the clustering of the field-of-views in the second feature space may be performed using a k-means clustering algorithm. Such a clustering algorithm is for example described in "https://de.wikipedia.org/wiki/k-means_clustering". It is a method of vector quantization that aims to partition n observations into k clusters, in which each observation belongs to the cluster with the nearest mean, also referred to as cluster centre.

According to an embodiment, the performance index for each combination of Bl-shim and field-of-view may be done by simulating a magnetization distribution or flip angle distribution from each Bl-shim in the field-of-view, and comparing the simulating distribution with a target magnetization or flip angle distribution, respectively, in particular by calculating a root-mean-square deviation, as described above. The magnetization distribution may mean the transversal magnetization which would be generated when applying each B1-shim to the respective field-of-view with its respective B1-maps. The magnetization may be complex, i.e., having a magnitude and phase. Instead of the magnetization, one may also work with the flip angle distribution, wherein the flip angle may be a real value for each pixel or voxel within the field-of-view.

According to an embodiment, the method comprises calculating a set of optimized initial Bl-shims, in particular one for each group of field-of-views mentioned in step (c) and (d). For example, the method may be used to calculate a set of 10 to 80, preferably 20 to 60 optimized initial Bl-shims. Thereby, one has a selection of different starting points for the B1-shimming during the MR measurement.

The method also comprises a method of finding the ideal starting point for B1-shimming during a MR measurement, from the set of optimized initial Bl-shims. Preferably, this selection method uses the small angle approximation for all flip angles. Thereby, the calculation is simplified, but the calculation is sufficiently precise to find differences between the various initial Bl-shims and allows to define a matrix for each field-of-view, and to estimate the magnetization distribution for the various optimized initial Bl-shims using a simple matrix multiplication, as described in the following in more detail.

In particular, the method may comprise a shimming method for performing B1-shimming during a magnetic resonance measurement on a field-of-view within the body part of a subject by means of a multi-element transmit coil, the shimming method comprising the steps of:

(i) receiving a set of optimized initial Bl-shims, which have been calculated by the method of one of the claims 1 to 7,
(ii) measuring Bl-maps of the body part;
(iii) using the Bl-maps for calculating the magnetization distribution resulting from the combination of each of the set of optimized initial Bl-shims with the field-of-view and storing the result in a magnetization matrix;
(iv) for each of the set of optimized initial Bl-shims, calculating a term comprising a parameter comprising a comparison of the magnetization matrix with a target magnetization distribution, a parameter comprising the minimal magnetization or flip angle within the magnetization matrix, and optionally a parameter comprising the phase rotation of the magnetization matrix;
(v) selecting the optimized initial Bl-shim for which the term calculated in step (ii) is at an extremum, as the starting point of a B1-shimming optimization.

This part of the method may be performed online, i.e., once the subject or patient has been positioned within the MRI system because it requires Bl-maps of the body part on which the MR measurement is to be performed. These Bl-maps are then used for calculating the magnetization distribution resulting from the combination of each of the set of optimized initial Bl-shims with the field-of-view, which is to be measured, and storing the results in a magnetization matrix. The "magnetization" may be the complex transverse magnetization which would be obtained when applying a certain RF pulse using the optimized initial Bl-shim. Alternatively, the "magnetization" may also mean the flip angle, or alternatively the Bl-field, which is obtained when using the optimized initial Bl-shim on the multi-element transmit coil from which the Bl-maps have been measured. Magnetization, flip angle and Bl-field all describe the same effect, therefore in this text, the "magnetization distribution" may also mean the flip angle distribution or the Bl-field distribution generated by the multi-element transmit coil. This magnetization distribution is stored in a magnetization matrix in step (iii) for each optimized initial Bl-shim. The "matrix" may mean that each pixel in the field-of-view is assigned one element in the magnetization matrix. The pixel size within the magnetization matrix may be as large as in a MR image. Alternatively, the pixel size may be somewhat larger in order to simplify the calculation, e.g., using a pixel size of 2 to 4mm. These matrices may then be used to calculate a term comprising a comparison of the magnetization matrix with a target magnetization distribution (or target flip angle distribution), the minimal flip angle within the magnetization matrix, and optionally the phase rotation of the magnetization matrix.

The comparison of the magnetization matrix with a target magnetization distribution may comprise calculating the root-means-square deviation (RMSE) between the magnetization matrix and the target magnetization distribution, as described herein. In particular, the corresponding parameter may be the normalized RMSE. Evidently, this RMSE should be as small as possible in order to find the best starting point among the set of optimized initial Bl-shims. Further, the term may comprise the minimum flip angle within the magnetization matrix, but in a way that of course those optimized initial Bl-shims are preferred, where the minimal flip angle is as large as possible. The minimal flip angle within the magnetization matrix characterizes possible "holes" in the magnetization distribution and thus in the final MR image. Therefore, magnetization matrices which have some local areas where the magnetization or flip angle is very small, are a less preferred starting point. For example, the parameter may comprise one minus the normalized minimal magnetization. According to an embodiment, the minimal magnetization matrix excludes up to 5%, more preferred up to 2%, most preferred up to 1% of the magnetizations, in order to avoid possible outliers. As an optional third parameter, the term may comprise a parameter including the phase rotation of the magnetization matrix. This may be calculated using the curl of the vector field which represents the magnetization matrix.

This term is calculated for each of the set of optimized initial Bl-shims, and that Bl-shim for which the term is at an extremum, in particular at a minimum, is selected as the starting point of a B1-shimming optimization. This B1-shimming may then be performed again using a known optimization algorithm, e.g., a gradient descent method such as the inner points method. However, by using a very good initial Bl-shim, it has been found that the B1-shimming optimization produces much more robust results than the state of the art. Further, the shimming method can be performed very quickly, e.g., in less than 1 second, during the MR examination while the patient is in the MR scanner. Time is of course very important during the MR examination itself.

According to an embodiment, the term of which the extremum, in particular the minimum, is taken, comprises the root-mean-square deviation between the magnetization matrix and a target magnetization distribution. The RMSE may in particular be the NRMSE. The RMSE may be weighted in the term.

Also, the minimum magnetization within the magnetization matrix may be weighted by a pre-determined weight, as may be the term including the phase rotation of the magnetization matrix. Thus, the term calculated in step (iv) may be a weighted sum of a parameter comprising the root-mean-square deviation between the magnetization distribution and a target magnetization distribution, a term including the minimum flip angle within the magnetization matrix, and optionally a term including the phase rotation of the magnetization matrix.

The invention is also directed to a computer program including a program code which causes a computer to carry out the method according to an embodiment of the invention, when the computer program is executed on a computer. The computer may be a stand-alone computer or a processing unit, such as a CPU, GPU, which may be part of a PC, main frame computer, cloud computer, server, or of course part of a control computer of an MR system. This is in particular true for the shimming method, which is preferably carried out during an MR examination.

According to a further aspect, the invention relates to a non-transient computer readable medium comprising a computer program with instructions, which when carried out on a computer, will cause the computer to carry out the method according to an embodiment of the invention. The computer-readable medium may be any digital storage medium, in particular an optical or magnetic storage medium, or a solid-state digital storage medium, such as an SD-card, SSD-card, USB-stick, hard disc or cloud solution.

The invention is further directed to a Bl-shim design unit configured for calculating at least one optimized initial B1-shim for a magnetic resonance imaging measurement on a field-of-view within a body part of a subject, wherein a Bl-shim comprises a vector of complex Bl-shim coefficients, each coefficient representing a scaling factor for one element of a multi-element transmit coil which is to be used in the magnetic resonance measurement, the Bl-shim design unit comprising: a data interface configured for receiving a set of previously measured Bl-maps of the multi-element transmit coil for one or several body parts of various test subjects, and for outputting the optimised initial Bl-shim; a calculating unit configured for performing the method of calculating at least one optimized initial Bl-shim according to an embodiment of the invention. In particular, the B1 shim design unit is configured to carry out the method according to the invention.

The Bl-shim design unit may operate offline, i.e., it does not have to be in direct connection to an MRI system, since it requires only Bl-maps measured previously from one or several (different) body parts of various test subjects. The rest of the method for calculating one or a set of optimized initial Bl-shims is performed by simulation, as described herein. The output is at least one optimized initial Bl-shim. Such initial Bl-shim may be used in the shimming method, or may be used by a control unit for a magnetic resonance imaging system, which is also part of the invention. The control unit is configured to carry out at least the B1-shimming method described herein. According to an embodiment, the control unit may also be configured to calculate the at least one optimized initial Bl-shim, i.e., it may be the Bl-shim design unit. Both the Bl-shim design unit and the control unit may comprise a processing unit, e.g., a CPU or a gradient coil system.

Finally, the invention is directed to an MRI system comprising such control unit. The MRI system may further comprise a main magnet, a gradient coil system and of course a multi-element transmit coil, as well as other elements known in the art.

All embodiments, features and advantages of the method according to the invention also apply to the computer program, the non-transient computer readable medium, the Bl-shim design unit, the control unit and the MRI system of the invention, and vice versa.

The invention is now described by means of embodiments with reference to the attached drawings, in which:

Fig. 1     is a schematic representation of an MRI apparatus according to an embodiment of the invention;

Fig. 2     shows a Bl-map of a multi-element RF coil having 6 channels;

Fig. 3     shows a schematic cross section through the human head, demonstrating the position of 6 field-of-views;

Fig. 4     is a flow diagram of a method according to an embodiment of the invention;

Fig. 5     is a flow diagram of a method for selecting a suitable optimized initial Bl-shim during an MR measurement.

Similar elements are designated with the same reference signs in the drawings.

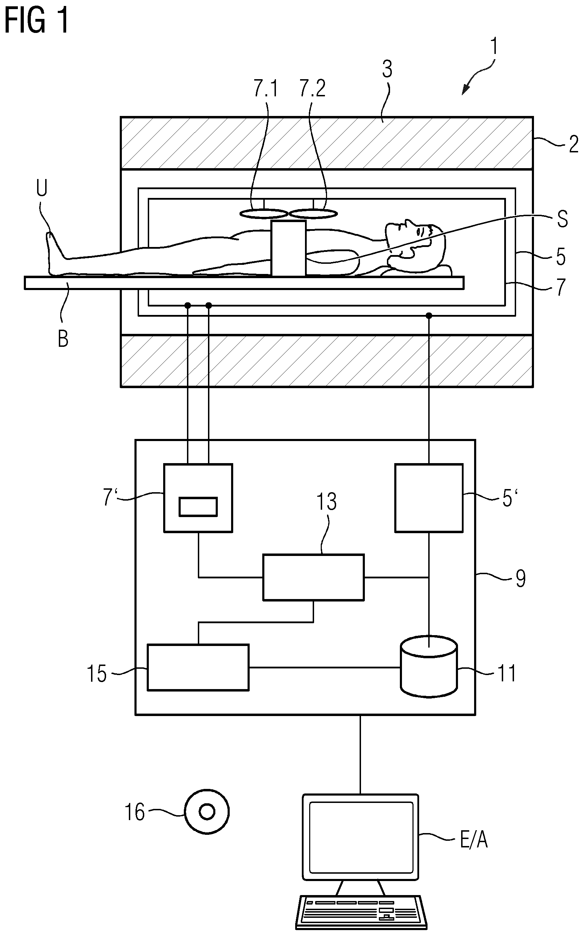

Fig. 1 schematically shows a magnetic resonance (MR) apparatus 1 according to an embodiment of the invention. The MR apparatus 1 has an MR data acquisition scanner 2 with a magnet 3 that generates a constant magnetic field (B0-field), a gradient coil arrangement 5 that generates gradient fields, one or several radio-frequency (RF) antennas 7 for transmitting and receiving RF signals, in particular Bl-fields, and a control computer 9 configured to perform the inventive method. The radio-frequency antennas may include a multi-element RF coil 7, such as a multi-channel coil array, comprising at least two coils, for example the schematically shown coils 7.1 and 7.2, which may be configured to transmit and/or receive RF signals (MR signals). In order to acquire MR data from an examination subject U, for example a patient or a phantom, the examination subject U is introduced on a bed B into the measurement volume of the scanner 2. The slab S is an example of a field-of-view within a body part of the examination subject, from which MR data can be acquired using a method according to an embodiment of the invention. The control computer 9 controls the MR apparatus 1, and can control the gradient coil arrangement 5 with a gradient controller 5' and the RF antenna 7 with a RF transmit/receive controller 7'. The RF antenna 7 has multiple channels corresponding to the multiple coils 7.1, 7.2 of the coil arrays, in which signals can be transmitted or received. The control computer 9 also has an imaging protocol processor 15 that determines the imaging protocols, including the measurement of B1 maps of the multi-element RF coil 7. A control unit 13 of the control computer 9 is configured to execute all the controls and computation operations required for acquisitions. Intermediate results and final results required for this purpose or determined in the process can be stored in a memory 11 of the control computer 9. Also, the at least one optimized initial B1-shim may be stored in the memory 11. A user can enter control commands and/or view displayed results, for example image data, an input/output interface E/A. A non-transitory data storage medium 16 can be loaded into the control computer 9, and may be encoded with programming instructions (program code) that cause the control computer 9, and the various functional units thereof described above, to implement any or all embodiments of the method according to the invention.

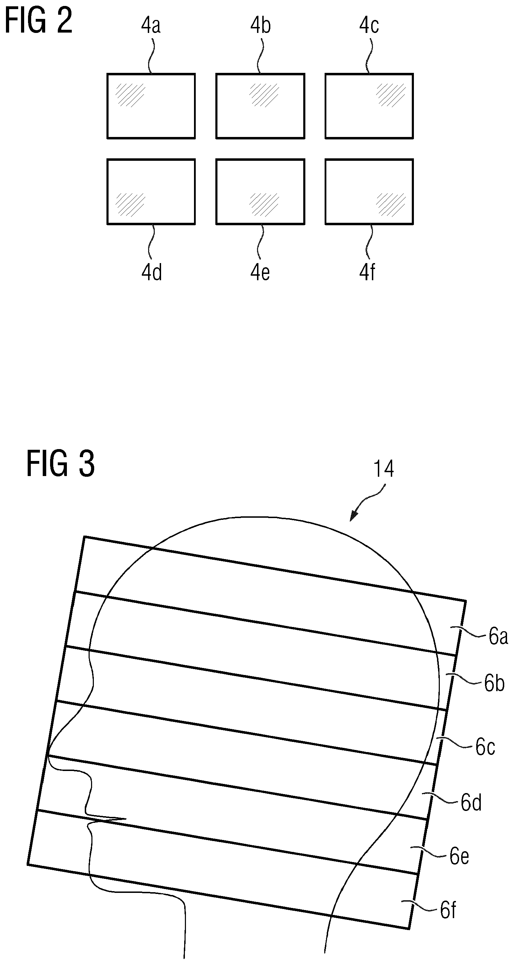

Fig. 2 illustrates a Bl-map which was measured for a multi-element RF transmit coil 7 for an individual patient. In this embodiment, the transmit coil 7 has 6 channels, i.e., 6 coil elements, wherein each coil element has a different sensitivity throughout the body part to be measured. These RF sensitivity maps are illustrated at 4a, 4b, 4c, 4d, 4e and 4f for each individual element 7,1. 7,2 of the coil 7, and are together referred to herein as Bl-map.

Fig. 3 illustrates a sagittal cross section through the human head 14, with 6 field-of-views 6a, 6b, 6c, 6d, 6e and 6f positioned therein in a typical almost transversal orientation. Each of these slabs or slices 6 may represent a field-of-view, for which a shim may be optimized. It is also possible that a field-of-view is only a part of a slice 6.

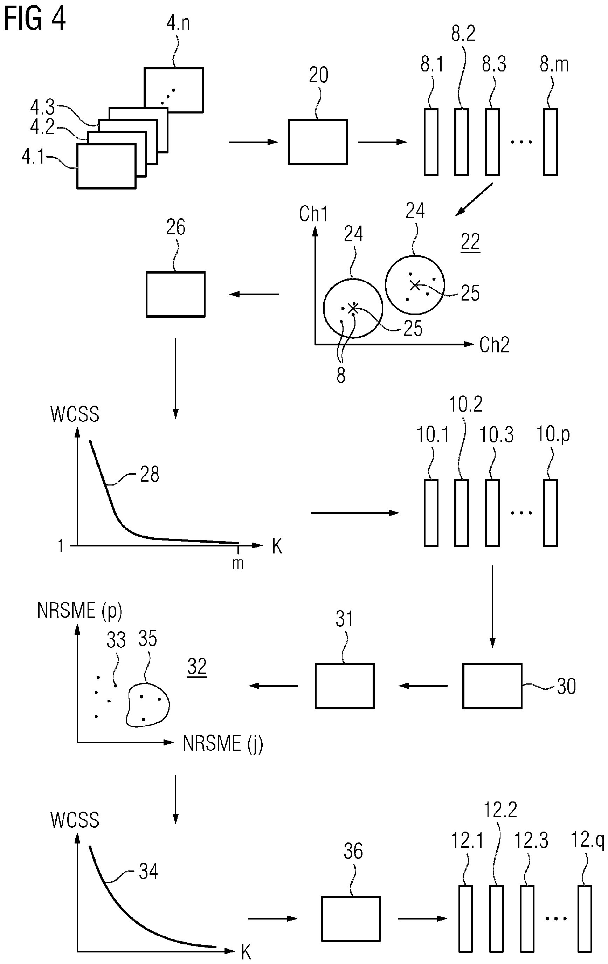

Fig. 4 illustrates a method according to an embodiment of the invention. The optimization method, which may be executed online, requires as input a number n of Bl-maps 4 acquired from various patients during different previous MR measurements. They may be all from the same body part, e.g. all from the human head, or alternatively from a variety of body parts and are illustrated at 4.1, 4.2, 4.3, ..., 4.n. In step 20, these different Bl-maps 4 are used in order to optimize shims for a number m different field-of-views within these Bl-maps, using a known optimization algorithm, e.g., a gradient descent method such as disclosed in Majewski. The result of this B1-shimming is a number m of Bl-shims 8, wherein each Bl-shim is essentially a vector of complex Bl-shim coefficients. Preferably, Bl-shims 8 are optimized for several field-of-views in several different orientations (e.g., transversal, coronal, sagittal). Preferably, circularly polarized Bl-fields or shims are used as starting values.

The thereby obtained Bl-shims 8 are then arranged in a first feature space illustrated at 22, wherein the coordinate axes of this feature space 22 are the real and imaginary parts of the shim coefficients, here illustrated as Ch1 (channel 1) and Ch2 (channel 2). In reality, each channel is represented by a complex shim coefficient, so there will be twice as many dimensions of this feature space 22 as channels or elements of the multi-element transmit coil 7. In the first feature space 22, each shim 8 is represented by one data point.

In a next step 26, k-means clustering is applied to the feature space 22 in order to divide the optimized Bl-shims 8 into several clusters 24. In order to find a suitable number of clusters, one may calculate the within-cluster-sum-of-squares (WCSS) value 28 as a function of the number of clusters k, wherein the maximum number of clusters is m, the number of Bl-shims. The WCSS value is a measure for how similar all B1-shims within one cluster are. Alternatively, the number of clusters may be pre-determined. Once a suitable number of clusters has been defined, a clustering may be carried out to define which Bl-shims belong to each cluster 24. In a next step, for each cluster 24 a centre of gravity (also referred to as midpoint) 25 is calculated. The centres of gravity 25 together may be regarded as covering a subspace of all possible Bl-shims, in which possible useful Bl-shims are contained. The centres of gravity 25 are then each regarded as a Bl-shim by themselves, resulting in a set of average Bl-shims 10.1, 10.2, 10.3, ..., 10.p, wherein p is the number of clusters.

In step 30, these average Bl-shims 10 are then used as starting points for an individual optimization on the various field-of-views, which have also been used in step 20. The result of this optimization 30 is then evaluated in step 31 by calculating the normalized root-mean-square deviations for each field-of-view. This may be done by simulating a flip angle distribution or Bl-field distribution or magnetization distribution for each field-of-view in step 31, and comparing this with the target flip angle or Bl-field (the magnitude thereof) or the magnetization (magnitude thereof). A suitable measure for the deviation may be the NRMSE. From these NRMSE values, a second feature space 32 is defined, in which each individual field-of-view is represented as one data point 33, and the coordinate axes or bases of this feature space 32 are the different NRMSE values of the different candidate shims 10. In this feature space, a further k-means clustering is carried out, wherein the different field-of-views are grouped into clusters 35. This may again be done by calculating the WCSS value for the number of clusters k, which may for example be 40. By this clustering, those field-of-views may be grouped together, for which a similar Bl-shim works similarly well (or less well).

In a next step 36, a final B1-shimming/optimization is carried out, wherein the field-of-views for each cluster are grouped together, i.e. the Bl-shim is optimized not for one field-of-view, but for several vield-of-views simultaneously. This may be done for all field-of-views within one cluster, or only those which are closest to the centre of gravity of the cluster 35, wherein the Euclidean distance may be used. The B1-shimming is done by using some or all of the Bl-shims 8 (which had been used to define the first feature space) as starting value for the optimization, and again evaluating the quality of the resulting Bl-shim, for example by calculating an NRMSE as described above. The Bl-shim resulting from the optimization and having the lowest NRMSE for each group of field-of-views is then taken as one optimized initial B1-shims 12.

The different clusters 35 represent the different Bl-field distributions, for which one has to optimize. By optimizing for several field-of-views (possibly in different orientations) together, one may find more robust optimized initial Bl-shims 12 than in a purely individual optimization, in which "holes" in the magnetization distribution may more easily occur. The result of this optimization 36 is thus a set of optimized initial Bl-shims 12, one for each cluster 35.

This method may be carried out for each individual magnetic resonance imaging system 1, e.g., during its setup, and the set of optimized initial Bl-shims 12 may be stored for later use during an MR measurement. Alternatively, they may be predefined for each type of MRI system 1 and multi-element RF coil 7.

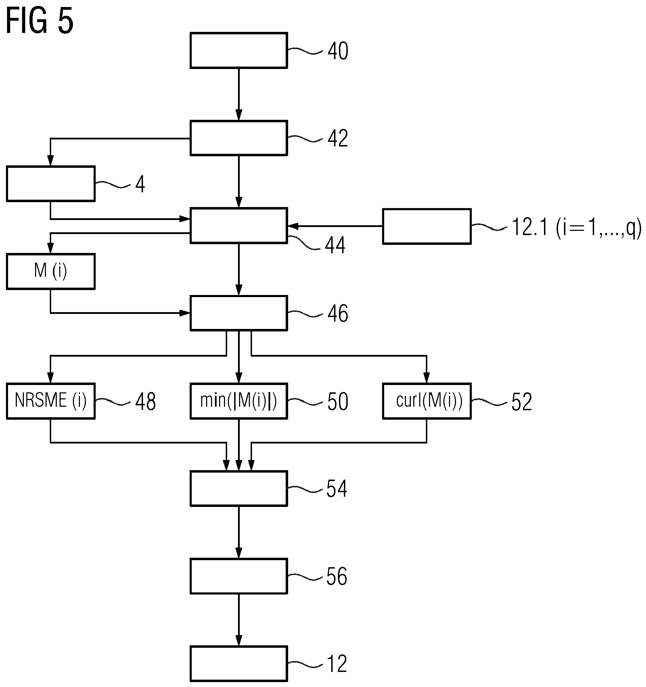

Fig. 5 illustrates a method which may be carried out during an MR measurement to find the one optimized initial Bl-shim 12 which is to be used as starting value for the B1-shimming in this particular examination. In step 40, the patient is placed in the sensitive area of the MRI system. In step 42, an individual Bl-map 4 of the region of interest within the body part to be measured in this particular MRI measurement is acquired. The actual Bl-map in this MRI measurement is used in step 44, as well as the set of optimized initial B1-shims 12.i, wherein i may be 1, 2, 3, ..., q, which are the optimized initial Bl-shims stored for this MRI system. In step 44, a magnetization matrix $\mathbf{M}$ (i) is calculated for each optimized initial Bl-shim. In this calculation, a small angle approximation may be used for all flip angles. This is sufficient to evaluate the relevant differences between several shims and opens the possibility to define a matrix $\mathbf{M}$ for each field-of-view, and to then estimate the different B1-distributions for different Bl-shims by a simple matrix multiplication. From these magnetization distributions, which are stored in the magnetization matrices $\mathbf{M}$ (i), 3 parameters are calculated in step 46, wherein the third parameter 52 is optional. These parameters are the NRMSE 48, the minimal flip angle 50 and the rotation 52. These 2 or 3 parameters are calculated for each field-of-view and added together in a weighted summation in step 54. In step 56, the shim 12 with index i is identified, for which the weighted sum of these 3 terms is the smallest of all shims. This process may be represented by the following formula:

$$\underset{i}{\arg\min}\left(\left(\frac{\mathrm{NRMSE}(i)}{\underset{\forall i}{\max}(\mathrm{NRMSE}(i))}\right) + w_{\mathrm{minFA}}\left(1 - \frac{\min(|\mathbf{M}(i)|)}{\underset{\forall i}{\max}(\min(|\mathbf{M}(i)|))}\right) + w_{\mathrm{phase\,rot}}\frac{\left|\mathrm{curl}\left(\begin{array}{c}\mathrm{Re}(\mathbf{M}(i))\\ \mathrm{Im}(\mathbf{M}(i))\end{array}\right)\right|_1}{\underset{\forall i}{\max}\left|\mathrm{curl}\left(\begin{array}{c}\mathrm{Re}(\mathbf{M}(i))\\ \mathrm{Im}(\mathbf{M}(i))\end{array}\right)\right|_1}\right)$$

in which $\mathbf{M}$ represents the transverse magnetization in each voxel in the particular field-of-view as a matrix, equivalent to a Bl-field distribution or flip angle plus phase, curl represents the rotation operator, and $w_{\mathrm{minFA}}$ is a weight for the minimal flip angle and may be between 0.5 and 2, for example 1.2, and the weight for the phase rotation is $w_{\mathrm{phase\,rod}}$, which may be between 1 and 2, for example 1.8. Please note that each of the 3 parameters is normalized, e.g., the NRMSE is divided by the maximum NRMSE of all shims with index i, so that the maximum value of this term is 1. In the second term, the minimum flip angle is divided by the minimum flip angle across all magnetization matrices with index i. Please not that in this case, the normalized minimum flip angle is subtracted from 1, so that this term is at a minimum if the minimum flip angle is at its maximum. In an embodiment, the minimum in each magnetization matrix is taken to be not the absolute minimum, but the minimum magnetization under exclusion of the lowest 1 to 5, preferably 2 percentiles in order to exclude possible outlying values. Also, the third term is normalized by dividing the rotation within each magnetization matrix by the maximum rotation across all magnetization matrices. The second parameter and also the third parameter is responsible for avoiding "holes" in the magnetization matrix and thus in the final image, since these often correlate with a strong phase rotation in the facility of the hole. The weight may be varied for different RF coils, and for the different body parts. The optimal weight parameters may also vary with the respective shim. The output of this method is one optimized initial BI-shim 12, which is then used to individually optimize the BI-shim for this particular field-of-view.

By the process in Fig. 5, one can very quickly find the optimal BI-shim for each field-of-view and use that as a starting value for the individual optimization. The time frame for finding the best initial BI-shim is mostly less than a second, in particular between 0.1 and 1 seconds, more preferably between 0.2 and 0.8 seconds.

Therefore, the invention provides a method to reach much improved BI-shims, and in particular avoids "holes" in the final image. The processing is mostly done in an offline step, which can be carried out beforehand and only once for each multi-element RF coil. Possibly, a set of optimized initial BI-shims may be provided for different body parts in combination with a certain multi-element RF coil. During the actual MR measurement, after carrying out the selection method for selecting the best initial BI-shim, a state-of-the-art shimming optimization algorithm can be used, and still much improved B1-shimming results are obtained. This so-called "cluster starting point" furnishes better homogeneity and higher flip angles, especially in those field-of-views in the lower part of the head, which are otherwise somewhat impaired by air inclusions in the throat area.

Independent of the grammatical term usage, individuals with male, female or other gender identities are included within the term.

## Claims

1.  A method for calculating at least one optimized initial BI-shim (12) for a magnetic resonance measurement,

    wherein a BI-shim comprises a vector of complex BI-shim coefficients, each coefficient representing a scaling factor for one element of a multi-element transmit coil which is to be used in the magnetic resonance measurement,
    the method comprising the steps of:

    (a) receiving a set of previously measured BI-maps (4) of the multi-element transmit coil (7) for one or several body parts (14) of various test subjects,
    (b) calculating (20) a set of BI-shims (8) for a plurality of different field-of-views (6) in the one or several body part(s) of the various test subjects from the previously measured BI-maps (4) by means of an optimization algorithm,
    (c) identifying which BI-shim has the best performance for a group of field-of-views by using the previously measured BI-maps (4), and
    (d) optimizing (36) the BI-shim for that group of field-of-views to obtain an optimized initial BI-shim (12).

2.  The method of claim 1, wherein the step (c) of identifying which BI-shim has the best performance for a group of field-of-views by using the previously measured BI-maps (4) comprises the steps of:

    (c1) clustering the BI-shims (8) calculated for the plurality of different field-of-views and identifying an average BI-shim (25, 10) for each cluster;
    (c2) applying (30) the average BI-shims (10) to each of the plurality of field-of-views and calculating (31) a performance index for each combination of average BI-shim (10) and field-of-view using the previously measured B1-maps;
    (c3) clustering (32) the field-of-views (33) according to their performance indices to identify groups of field-of-views (35) which perform similarly well with a similar BI-shim, and
    wherein the step (d) comprises optimizing the BI-shim for each group of field-of-views (35), to obtain an optimized initial BI-shim (12).

3.  The method of claim 1 or 2, comprising the steps of performing a clustering of the BI-shims (8) calculated in step (b) and identifying an average Bl-shim (12) for each cluster by

(c1.1) representing the set of Bl-shims (8) calculated in step (b) in a first feature space (22), wherein the dimensions of the feature space are the complex shim coefficients of each Bl-shim,
(c1.2) performing a cluster analysis (26) of the Bl-shims (8) in the first feature space (22) and calculating a midpoint (25) of each cluster, each midpoint being the average Bl-shim (12) for that cluster.

4. The method of claim 2 or 3, comprising the steps of clustering the field-of-views according to their performance indices and optimizing the Bl-shim to obtain an optimized initial Bl-shim (12) for this group of field-of-views by

(c3.1) representing the performance indices (33) calculated in step (c2) in a second feature space (32), in which each field-of-view is represented by one data point (33), and the dimensions of the second feature space (32) are the performance indices of each average B1-shim;
(c3.2) performing a cluster analysis on the second feature space (32), and determining the groups of field-of-views which are closest to the centre of each cluster (35) ;
(d.1) for each group of field-of-views, calculating an optimized Bl-shim using an optimization algorithm;
(d.2) providing the optimized Bl-shims as optimized initial Bl-shims (12) for the field-of-views within the group (35) of field-of-views.

5. The method of one of the preceding claims, wherein the clustering (26) of the Bl-shims in the first feature space and/or the clustering (32) of the field-of-views in the second feature space is performed using a k-means Clustering algorithm.

6. The method of one of claims 2 to 5, which comprises calculating a performance index for each combination of average Bl-shim (10) and field-of-view by simulating a magnetization distribution or flip angle distribution from each Bl-shim and comparing the simulated distribution with a target magnetization or flip angle distribution.

7. The method of claim 6, wherein comparing the simulated distribution with the target magnetization or flip angle distribution comprises calculating a root-mean-square deviation (NRMSE).

8. The method one of the preceding claims, which comprises calculating a set of optimized initial Bl-shims (12).

9. The method one of the preceding claims, which comprises calculating one optimized initial Bl-shim for each group of field-of-views.

10. The method of one of the preceding claims, the method comprising a shimming method for performing B1-shimming during a magnetic resonance measurement on a field-of-view within a body part of a subject by means of a multi-element transmit coil, the shimming method comprising the steps of:

(i) receiving a set of optimized initial Bl-shims (12), which have been calculated by the method of one of the claims 1 to 7,
(ii) measuring (42) Bl-maps (4) of the body part;
(iii) using the Bl-maps (4) for calculating the magnetization distribution resulting from the combination of each of the set of optimized initial Bl-shims (12) with the field-of-view and storing the result in a magnetization matrix;
(iv) for each of the set of optimized initial Bl-shims (12), calculating (46) a term comprising a parameter (48) comprising a comparison of the magnetization matrix with a target magnetization distribution, and a parameter (50) comprising the minimal magnetization or flip angle within the magnetization matrix;
(v) selecting the optimized initial Bl-shim for which the term calculated in step (ii) is at an extremum, as the starting point of a B1-shimming optimization.

11. The method of claim 10, wherein the term calculated in step (iv) comprises a parameter (52) comprising the phase rotation of the magnetization matrix.

12. The method of claim 10 or 11, wherein the term calculated in step (iv) comprises a parameter comprising the root-mean-square deviation between the magnetization matrix and a target magnetization distribution.

13. The method of claim 11 or 12, wherein the term calculated in step (iv) is a weighted sum of a parameter comprising the root-mean-square deviation (48) between the magnetization distribution and a target magnetization distribution, and a parameter (50) including the minimum flip angle within the magnetization matrix.

**14.** The method of claim 11 or 12, wherein the term calculated in step (iv) is a weighted sum of a parameter comprising the root-mean-square deviation (48) between the magnetization distribution and a target magnetization distribution, a parameter (50) including the minimum flip angle within the magnetization matrix, and a parameter (52) including the phase rotation of the magnetization matrix.

**15.** Computer program including program code which causes a computer to carry out the method according to any one of claims 1 to 14, when the computer program is executed on a computer.

**16.** Non-transient computer-readable medium comprising a computer program with instructions which, when carried out on a computer, will cause the computer to carry out the method according to any one of the claims 1 to 14.

**17.** A Bl-shim design unit (13) configured for calculating at least one optimized initial Bl-shim for a magnetic resonance imaging measurement on a field-of-view within a body part of a subject,
wherein a Bl-shim comprises a vector of complex Bl-shim coefficients, each coefficient representing a scaling factor for one element of a multi-element transmit coil which is to be used in the magnetic resonance measurement, the Bl-shim design unit comprising:

- a data interface configured for receiving a set of previously measured Bl-maps of the multi-element transmit coil for one or several body parts of various test subjects, and for outputting the optimised initial Bl-shim;
- a calculating unit configured for performing the method of one of claims 1 to 8.

**18.** Control unit (15) for a magnetic resonance imaging system, wherein the control unit is configured to carry out the method according to any one of the claims 1 to 8, and/or is configured to control a magnetic resonance imaging system to carry out the method according to any one of claims 9 to 14.

**19.** Magnetic resonance imaging system (1) comprising a control unit (15) according to claim 18.

# FIG 1

FIG 2

4a   4b   4c

4d   4e   4f

FIG 3

14

6a
6b
6c
6d
6e
6f

# FIG 4

# FIG 5

Europäisches
Patentamt

European
Patent Office

Office européen
des brevets

## EUROPEAN SEARCH REPORT

Application Number

EP 24 16 2192

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/011155 A1 (ITO KOSUKE [JP] ET AL) 11 January 2018 (2018-01-11) * the whole document * | 1-19 | INV. G01R33/565 G01R33/561 G01R33/56 |
| A | ZENG QI ET AL: "Impact of RF Shimming on RF-Induced Heating Near Implantable Medical Electrodes in a 3T MRI Coil", IEEE TRANSACTIONS ON ELECTROMAGNETIC COMPATIBILITY, IEEE SERVICE CENTER , NEW YORK , NY, US, vol. 62, no. 1, 13 November 2018 (2018-11-13), pages 52-64, XP011772839, ISSN: 0018-9375, DOI: 10.1109/TEMC.2018.2877526 [retrieved on 2020-02-14] * the whole document * | 1-19 | |
| A | EP 3 153 874 A1 (COMMISSARIAT L ENERGIE ATOMIQUE ET AUX ENERGIES ALTERNATIVES [FR]) 12 April 2017 (2017-04-12) * the whole document * | 1-19 | |
| A | WO 2011/033402 A1 (KONINKL PHILIPS ELECTRONICS NV [NL]; HARVEY PAUL R [NL] ET AL.) 24 March 2011 (2011-03-24) * the whole document * | 1-19 | TECHNICAL FIELDS SEARCHED (IPC) G01R |
| A | EP 3 594 710 A1 (COMMISSARIAT ENERGIE ATOMIQUE [FR] ET AL.) 15 January 2020 (2020-01-15) * the whole document * | 1-19 | |

The present search report has been drawn up for all claims

| Place of search | Date of completion of the search | Examiner |
|---|---|---|
| Munich | 31 July 2024 | Durst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 431 970 A1**

## ANNEX TO THE EUROPEAN SEARCH REPORT
## ON EUROPEAN PATENT APPLICATION NO.

EP 24 16 2192

31-07-2024

| Patent document cited in search report | Publication date | Patent family member(s) | Publication date |
|---|---|---|---|
| US 2018011155 A1 | 11-01-2018 | JP 6618988 B2 | 11-12-2019 |
| | | JP WO2016143460 A1 | 21-12-2017 |
| | | US 2018011155 A1 | 11-01-2018 |
| | | WO 2016143460 A1 | 15-09-2016 |
| EP 3153874 A1 | 12-04-2017 | EP 3153874 A1 | 12-04-2017 |
| | | US 2018252788 A1 | 06-09-2018 |
| | | WO 2017060142 A1 | 13-04-2017 |
| WO 2011033402 A1 | 24-03-2011 | BR 112012005688 A2 | 30-05-2017 |
| | | CN 102498411 A | 13-06-2012 |
| | | EP 2478384 A1 | 25-07-2012 |
| | | JP 2013505046 A | 14-02-2013 |
| | | US 2012161766 A1 | 28-06-2012 |
| | | WO 2011033402 A1 | 24-03-2011 |
| EP 3594710 A1 | 15-01-2020 | EP 3594710 A1 | 15-01-2020 |
| | | JP 7317589 B2 | 31-07-2023 |
| | | JP 2020006162 A | 16-01-2020 |
| | | KR 20200006003 A | 17-01-2020 |
| | | US 2020011953 A1 | 09-01-2020 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K. MAJEWSKI.** Simultaneous optimization of radio frequency and gradient waveforms with exact Hessians and slew rate constraints applied to kT-points excitation. *Journal of Magnetic Resonance,* 2021, vol. 326, ISSN 1090-7807, 106941
- **A. HOYOS-IDROBO ; P. WEISS ; A. MASSIRE ; A. AMADON ; N. BOULANT.** On Variant Strategies to Solve the Magnitude Least Squares Optimization Problem in Parallel Transmission Pulse Design and Under Strict SAR and Power Constraints''. *IEEE Transaction on Medical Imaging,* March 2014, vol. 33 (3), 739-748
- **K. SETSOMPOP ; L.L. WALD ; V. ALAGAPPAN ; B.A. GAGOSKI ; E. ADALSTEINSSON.** Magnitude least squares optimization for parallel radio frequency excitation design demonstrated at 7 Tesla with eight channels. *Magn. Reson. Med.,* 2008, vol. 59 (4), 908-915

- **VAN DE MOORTELE P.F et al.** Calibration tools for RF shim at very high field with multiple element RF Coils: from ultra fast local relative phase to absolute magnitude B1+ mapping. *Proceedings of the 15th Annual Meeting of ISMRM, Berlin,* 2007
- **KAWIN SETSOMPOP.** Design Algorithms for Parallel Transmission in Magnetic Resonance Imaging. *Massachusetts Institute of Technology,* 2008
- **MAO W ; SMITH MB ; COLLINS CM.** Exploring the limits of RF shimming for high-field MRI of the human head. *Magn. Reson. Med,* 2006, vol. 56 (4), 918-922